# EUROPEAN PATENT SPECIFICATION

(11) **EP 4 163 221 B1**
(45) Date of publication and mention of the grant of the patent: **28.08.2024**
(21) Application number: 22208475.8
(22) Date of filing: 12.06.2018
(51) Int. Cl.: B65D 21/02, A01K 67/033, B65D 85/50

(54) **SPAWN STRUCTURE AND COMBINATION OF A CONTAINER AND AT LEAST ONE SPAWN STRUCTURE**
BRUTSTRUKTUR UND KOMBINATION EINES BEHÄLTERS UND MINDESTENS EINER BRUTSTRUKTUR
STRUCTURE DE FRAUDUL ET COMBINAISON D'UN RÉCIPIENT ET D'AU MOINS UNE STRUCTURE DE FRAUDUL

(30) Priority: 15.06.2017 NL 2019079
(43) Date of publication of application: 12.04.2023
(62) Divisional of application: 18740319.1
(73) Proprietor: Proti-Farm R&D B.V., 3852 AB Ermelo (NL)
(72) Inventor: STAAL, Simon, 2645 NK Delfgauw (NL); BOLIER, Lucas Jan, 25120 ED Den Haag (NL); DE BRUIN, Johan, 3882 RE Putten (NL)
(74) Representative: Santarelli

(56) References cited:
- EP-A1- 0 092 888
- WO-A2-2016/153340
- CN-U- 204 860 636

## Description

The first aspect of the present disclosure relates to an open topped, stackable, molded plastic container, which is generally rectangular with a bottom and first and second pairs of parallel upright walls joined to the bottom and at corners via corner structures.

From the prior art, stackable containers are well known. To be suitable for the purpose of breeding and rearing insects, a type of containers is desired which is stackable up to a height of at least 15 containers, and which can be used in a climate housing, having a temperature up to 40°C and a humidity of up to 90%.

According to this first aspect, the corner structure of the container comprises:
- a substantially diagonal and upright wall section which, seen in top view, extends diagonally between adjoining upright walls and blends into said upright walls to define the contour of a containment space of the container; and
- a top wall extending outward from an upper end of the diagonal and upright wall section; and
- a hollow tubular post extending vertically downward from said top wall, said hollow tubular post being spaced from said diagonal and upright wall section, wherein the top wall has a hole aligned with the hollow tubular post;
- wherein multiple, e.g. at least three, vertical stacking ribs are arranged circumferentially spaced about the hollow tubular post, each stacking rib having a vertical inner end joined to said hollow tubular post,
   o wherein at least one, preferably two, of said stacking ribs have a vertical outer end joined to said diagonal and upright wall section and/or to said adjoining upright wall section; and
   o wherein each of said stacking ribs has a lower end, said lower ends being located in a common plane;
wherein said tubular post has a protruding stacking pin portion extending downward beyond said common plane of the stacking rib lower ends; such that - in a stack - the pin portion of an upper container is received through the hole in the top wall of the corner structure of a lower container onto which said upper container is stacked.

This type of corner structure provides structural stability to stacks of at least 21 containers on top of each other, in combination with a relatively large containment space.

The open topped, stackable, plastic container is preferably made from polypropylene (PP), and is preferably monolithically injection molded.

In a stack is the pin portion of an upper container received through the hole in the top wall of the corner structure of a lower container onto which said upper container is stacked. The stacking rib lower ends of the upper container, located in a common plane, rest onto the top wall, adjacent the hole, of the lower container.

In embodiments, said stacking pin portion does not protrude downward beyond the bottom of the container (or terminates above the bottom) and the lower ends of the stacking ribs lie above the bottom. As a result, prior to stacking, a container rests on the floor with its bottom, and in a stack, the upper container nests in the lower container. Once stacked, the top wall of the lower container is adjacent the first and second pairs of parallel upright walls of the upper container.

In embodiments wherein the stacking pin does protrude downward beyond the bottom of the container, the container, prior to stacking, will rest onto these stacking pins. In embodiments with the lower ends of the stacking ribs above the bottom, the upper container will nest in the lower container. In embodiments wherein the lower ends of the stacking ribs lie below the bottom, in a stack, the upper container does not nest in the lower container but will be located at a distance above this lower container.

Advantageously, the upper ends of said stacking ribs join said top wall. This is in particular advantageous in view production of the container by injection moulding. The stacking ribs have an essentially rectangular cross-section. Also advantageous in view of the production is to design the rigs tapering in the direction of the stacking pin, e.g. to a thickness at the lower ends of 3,2 mm.

In embodiments, the corner structure further comprises quarter-circular skirt walls, extending (depending) downwards from an outward perimeter of the top wall.

Preferably at least one of said stacking ribs has an has an outer vertical end joined which is not joined to said diagonal and upright wall section and/or said adjoining upright wall section, but to said to said quarter- circular skirt wall.

Hence, the inner ends of each stacking rib are joined to the post, and preferably the upper ends of said stacking ribs join the top wall. At least one, preferably two, of said stacking ribs have an outer vertical end joined to said diagonal and upright wall section and/or said adjoining upright wall section. Possibly, one or more other stacking ribs have an outer vertical end joined to a quarter-circular skirt wall.

In embodiments, the bottom of the spawning container is provided with spawn structure clamps, to be used in combination with a spawn structure according to the second aspect of the present disclosure.

In embodiments, top flanges extend outward from an upper end of the upright walls. Advantageously, these top flanges and/ or the top walls are provided with one or more drainage holes. It is conceivable that the top flanges extend parallel to the bottom of the container, but it is also possible that parts of the upright wall are sloped, resulting in top flanges extending at an angle with respect to the bottom.

Advantageously, flange skirt walls depend downwards from the top flanges, essentially parallel to and at a distance from the respective first and second pairs of parallel upright walls. The skirt walls of top flanges extending parallel to the bottom and extending at an angle with respect to the bottom may blend into each other. Advantageously, the top flanges have a common width, preferably corresponding to the width of the top wall adjacent the upright walls. Preferably, at least one of the skirt walls is provided with a label, e.g. via in- mould labelling.

The first aspect of the disclosure also relates to the use of such a container in an insect breeding facility, e.g. for breeding and/ or rearing insects. Furthermore, the first aspect relates to a stack comprising at least 15, preferably at least 20, containers. Such a container may have a content weight of 15-20kg.

Further, the first aspect of the disclosure relates to a climate housing of an insect breeding facility, housing multiple stacks of inventive containers.

The second aspect of the disclosure, which is the subject matter of the present invention, relates a spawn structure and to the combination of an insect spawning container and at least one spawn structure.

Such a combination is known from WO2016/153340 of the same applicant, disclosing an insect spawning container to be used in an insect breeding facility for breeding insects of the type with crawling mother beetles having a protractab!e egg-laying tube, such as lesser mealworms or zophobas morios, in which insect spawning container at least one spawn structure is provided in which the mother insects will spawn their eggs, wherein the insect spawning container comprises a bottom, and the at least one spawn structure adjoins the bottom, the at least one spawn structure having a scalable face such that the mother beetles can crawl from the bottom onto and up along the scalable face of the spawn structure, and the at least one spawn structure comprising a multitude of crevices accessible from the scalable face, the crevices having dimensions tuned to the egg-laying tube of the mother beetles.

The aim of the second aspect is to provide an improved combination, providing an increased yield in an insect breeding process.

This is achieved by providing a combination according to the present invention, comprising:
- an insect spawning container adapted for use in an insect breeding facility for breeding insects of the type with crawling mother beetles having a protractable egg-laying tube, such as lesser mealworms or zophobas morios; wherein the insect spawning container comprises a bottom and first and second pairs of parallel upright walls;
- at least one spawn structure in which the mother insects will spawn their eggs, comprising a foldable member with a fold section provided centrally between, and via hinges connected to, a perforated left-hand section and a perforated right-hand section; the spawn structure further comprising a rigid plate;

wherein the foldable member and the rigid plate are movable with respect to each other between an open configuration and a closed spawning configuration; wherein in the closed spawning configuration the foldable member is folded such that the left-hand section and the right-hand section are parallel to each other, with the rigid plate sandwiched between the sections, creating a multitude of crevices between the rigid plate and inner faces of the folded member, the crevices having dimensions tuned to the egg- laying tube of the mother beetles; and in which spawning configuration outer faces of the folded member form two climbing faces;
wherein the spawn structure in the closed spawning configuration is positioned in the container such that the mother beetles are able to crawl from the bottom onto and up along the climbing faces of the spawn structure into the crevices; and wherein in the open configuration the foldable member is folded open allowing harvesting of the eggs. This is an advantageous configuration as folding a foldable member around the rigid plate allows the opening by a sideways movement of the foldable member, similar to the opening of a book, diminishing the risk of damaging the eggs, as no transversal relative sliding movement of the foldable member and rigid plate of the spawn structure is possible. Furthermore, the dimensions of the crevices are well-defined and reproducible. As a result, the yield is increased.

In a third aspect, not according to the present invention, this disclosure relates to a method for breeding insects of the type with crawling mother beetles having a protractable egg-laying tube, such as lesser mealworms or zophobas morios, comprising the steps of:
- providing a plurality of spawning containers in combination with spawn structures in the closed spawning configuration, as described above;
- providing adult insects including mother insects to the spawning containers;
and periodically repeating the steps of:
- removing a spawn structure from the spawning container, leaving the adult insects in the spawning container;
- folding open the foldable member of the spawn structure;
- removing the foldable member and/ or the rigid plate of the spawn structure holding the eggs, and allowing the eggs to hatch;
- providing an empty spawn structure in the closed spawning configuration in each spawning container.

This third aspect further relates to the use of the combination.

The crevices in the spawning configuration of the spawn structure have dimensions allowing the entry and passage of a protracted egg-laying tube of the beetle into the crevice, to deposit her eggs in the crevices onto the foldable member and/or rigid plate of the spawn structures during spawning, while prohibiting the entry of the mouth into the crevice, and thus preventing the beetles to eat the eggs.

The deposition of eggs involves positioning the egg-laying tube into the crevice, and sticking the eggs onto a part of the spawn structure. This can be both the foldable member and rigid plate of the spawn structure. In embodiments, one of the foldable member and rigid plate is made non-sticking, ensuring that all the eggs stick onto the other part of the spawn structure. Advantageously, the foldable member of the spawn structure is non- sticking, allowing all eggs to be spawned onto the rigid plate of the spawn structure.

In an open configuration the distance between the foldable member and rigid plate of the spawn structure is increased. In the open configuration, an enlarged area (free space) around the eggs is created, which is advantageous to optimize the hatching climate. The eggs are quite vulnerable and when hatching out, the baby larvae need more space to get out of the crevices. When the crevices are too small, risks are that the eggs become moist, and that the larvae stick to the walls of the crevice. It is conceivable that the enlarged area is created at the start of the hatching process, or only at the end after several days of hatching, or therebetween.

The enlarged area may also only be obtained for the purpose of harvesting the baby larvae. It is also conceivable that the enlarged area is obtained only for the purpose of cleaning the spawn structure.

Advantageously, the entire spawn structure is removed from the spawning container, and in a subsequent step the distance between the parts of the spawn structure is increased, allowing the foldable member and/or rigid plate of the spawn structure comprising the eggs to hatch, e.g. to be transported to the hatch area. In embodiments where one of the parts of the spawn structure is made non- sticking, allowing all eggs to be spawned onto the other part of the spawn structure, it is advantageous to allow the part of the spawn structure with the eggs sticking onto it to hatch, e.g. in a hatching chamber, while cleaning the other part of the spawn structure. After cleaning, this other part can be assembled with a new part without eggs to form a closed spawning configuration to be placed into the spawning container. Advantageously, the eggs stick onto the rigid plate of the spawn structure. Hence, while allowing a rigid plate with eggs to hatch, another rigid plate can be placed in the cleaned foldable member and positioned back into the spawning container.

Advantageously, the foldable member has a length in the direction of the fold section, essentially corresponding to the length of the rigid plate, and wherein the width of the left- hand and right-hand sections essentially correspond to the width of the rigid plate. With such tuned dimensions, in the spawning configuration the foldable member and rigid plate match onto each other allowing handling of the spawn structure as a single entity. Furthermore, such a configuration allows optimization of the amount of crevices between the foldable member and rigid plate in a single spawn structure. The rigid plate of the spawn structure advantageously has a length which is four times larger than its width. Accordingly, the foldable member of the spawn structure, prior to folding, has a length in the direction of the fold section that is roughly twice the width. For example, the length of the rigid plate of the spawn structure is 40-80 cm, and the height 10-20 mm. An advantageous thickness of the left-hand and right-hand sections of the foldable plate, as well as the rigid plate, is 2-5, preferably 3 mm.

The fold section has a width in the unfolded position in a range between a width essentially corresponding to the thickness of the second plate, and a width which is 1,1 - 2,5 times the thickness of the second plate.

The width of the fold section is e.g. dependent on the type of hinge. Advantageously, the foldable member of the spawn structure is provided with living hinges between the fold section on the one hand, and the left-hand and right-hand sections on the other hand. A living hinge is a thin flexible, or a so-called flexure bearing. The thickness of the foldable member at the location of such a living hinge may be reduced to less than 0,5, in particular 0,35mm. With such a living hinge, it is possible to have a width of the fold section essentially corresponding to the thickness of the second plate.

In embodiments, the fold section comprises a slit seat having a length corresponding to the length of the rigid plate, a width essentially corresponding to the thickness of the rigid plate, and a depth to accommodate the rigid plate. The depth of the slit is e.g. 5-15 mm, preferably around 10 mm. Advantageously, the width of the slit is tuned to the thickness of the rigid plate such that the rigid plate is clasped into the slit. In embodiments, the upper edges of the slit are provided with tilted guide surfaces to assist in guiding the rigid plate into the slit. With such a fold section comprising a slit, the fold section has a width in the unfolded position of about 2 times the thickness of the second plate. Also in such embodiments wherein the fold section comprises a slit, it is possible to provide a living hinge between the fold section and the left-hand and right-hand sections.

In embodiments, at least one of the hinges between the sections and the fold section is pre- stressed opposite the folding direction. As a consequence, upon folding the foldable member around the rigid plate of the spawn structure, the pre-stress has to be overcome to bring the left-hand section, the rigid plate and the right-hand section parallel to each other. This is in particular advantageous when the spawn structure is to be opened. With the eggs deposited onto the foldable member and/ or rigid plate of the spawn structure, the parts of the spawn structure may stick onto each other. With the pre-stressed hinge, the pre-stress assists in increasing the distance between the foldable member and rigid plate of the spawn structure, and overcoming the possible sticking force.

In embodiments, the ends of the inner faces of the foldable member opposite the fold section taper towards the outer faces. Possibly, instead thereof or in addition thereto the sides of the foldable member adjacent the fold section taper towards the outer faces. By allowing the ends to taper towards the outer faces, in the spawning configuration an opening slit is created, between the parallel left-hand section and the right-hand section on the one hand, and the rigid plate on the other hand. This opening slit facilitates the increasing of the distance between the foldable member and rigid plate of the spawn structure to the open configuration. Possibly, the opening slits allow the use of an opening tool to increase the mutual distance.

In embodiments, the bottom of the insect spawning container is provided with spawn structure clamps to receive the spawn structure in the spawning configuration and allow the spawn structure to be connected to the container.

Possibly, a spawn structure clamp comprises opposed protruding fingers, protruding from the bottom of the insect spawning container, between which a spawn structure receiving groove is provided. Possibly, the configuration of the opposed fingers is such that the bottom of the groove between the fingers is provided at a distance from the bottom of the spawning container, e.g. 2-4, preferably 3 mm. Advantageously, the fingers have tapering ends, to provide a guide surface for the spawn structure.

Preferably, the ends of the outer faces of the foldable member opposite the fold section are provided with complementary container connectors, allowing the spawn structure to be connected to the container in the spawning configuration. Advantageously, the configuration of the spawn structure clamps and the complementary container connectors is such that by connecting the spawn structure to the container, the position of the spawn structure in the container is defined. Possible container connectors are snap-fitments, protruding from the outer faces.

The spawn structure in the closed spawning configuration is positioned in the container such that the mother beetles are able to crawl from the bottom onto and up along the climbing faces of the spawn structure into the crevices. Possibly, the spawn structure is positioned adjoining the bottom of the container. It is also possible that the mother beetles crawl onto the climbing faces via the spawn structure clamps of the container.

In embodiments, the length of the spawn structure is between 70-100%, in particular 80- 90%, of that of the insect spawning container, and wherein preferably 2-10, in particular 4-6 spawn structure are provided in the longitudinal direction of the spawning container. With multiple of such elongated spawn structures in the spawning container, an optimum number of eggs is harvested. Advantageously, the spawn structures are provided along the sides of the spawning container, leaving a clear central longitudinal space in the spawning container. In this clear space, having a width of essentially 30-70%, in particular 40-60% of the width of the spawning container, the mother beetles are free to move around, possibly receive food, and provide ventilation. Optionally, the bottom of the spawning container is in this clear space provided with a perforated area to allow the removal of excrements.

According to the invention, in the closed spawning configuration the foldable member and rigid plate of the spawn structures are positioned with respect to each other such that a multitude of crevices is created therebetween. The foldable member is provided with perforated sections, i.e. sections provided with cut-aways, wherein crevices are at least created between an outer contour of the perforation and the rigid plate of the spawn structure.

For breeding lesser mealworms the width (w) of the crevices is 0,2-1 ,2 mm, while for breeding zophobas morios the width of the crevices is 0,2-1 ,8 mm. The depth (d) of the crevices may vary between 0,5-10,0 mm.

For a spawn structure having a length of 40-80 cm, and a height 10-20 mm, advantageously the left-hand section and the right-hand section are each provided with 250-400 perforations, in particular 300-350. It is envisaged that aspects of the first aspect of the disclosure can be combined with the second aspect, and vice versa.

The invention is further elucidated in relation to the drawings, in which:
Fig. 1 a represents a side view of a stack of two containers according to the first aspect of the present disclosure;
Fig. 1b represents a perspective top view of a container according to the first and second aspect of the disclosure;
Fig. 2a represents a perspective bottom view of a corner structure of the container of figs. 1 a and 1 b;
Fig. 2b represents a detailed side view of a corner structure of the container of figs. 1 a and 1 b;
Fig. 2c represents a cross-section of the corner structures of two stacked containers of fig. 1 a;
Fig. 2d represents a bottom view of the corners structure of the container of figs. 1 a and 1 b;
Fig. 3a-3e represent a foldable member of a spawn structure of a combination according to an embodiment of the invention, respectively in a perspective view from below, a top view, a side view, a perspective view from above and a detail thereof;
Fig. 4 represents a perspective view onto a spawn structure according to an embodiment of the invention in the open configuration;
Figs. 5a and 5b represent perspective views onto the entire spawn structure according to an embodiment of the invention in the closed configuration and a detail thereof;
Figs. 6a-6d represent details of the fold section of the foldable member of the spawn structure according to an embodiment of the invention;
Figs. 7a, 7b and 8a-8d represent detailed perspective views of a spawn structure clamp and a complementary container connector on the foldable plate;
Figs. 9a and 9b represent a perspective view and a top view of a combination of an insect spawning container and a spawn structure according to an embodiment of the invention.

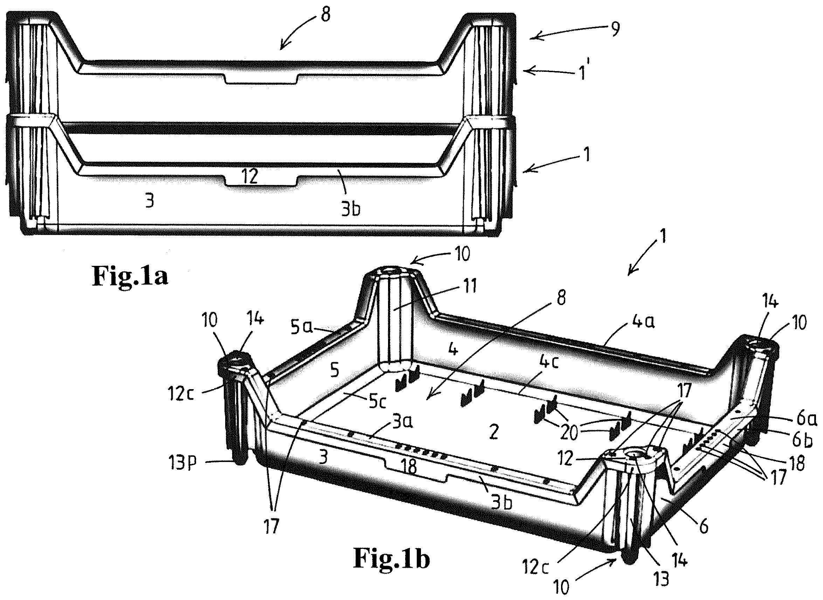

In figs. 1a -2d, an open topped, stackable, plastic container 1 of the first aspect of the disclosure is shown, with details of an inventive corner structure 10. In figs. 1 a and 2c, two stacked containers 1 and 1 ' are shown, wherein same parts are given same reference numerals to which an apostrophe (') has been added.

The container is preferably a monolithically injection molded container, preferably made from PP. The container 1 is generally rectangular with a bottom 2 and first and second pairs of parallel upright walls 2, 3, 4, 5 joined to the bottom 2 and at corners via inventive corner structures 10, which will be discussed in detail below.

Here, at a bottom end, the upright walls 2, 3; 4, 5 join (and blend into) the bottom via curved sections 2c, 3c; 4c, 5c. A common wall thickness for this type of containers is 2-3,5 mm.

The dimensions of such a container are e.g. a length of 700-800 mm, a width of 500-600 mm and a height of 100-200 mm. With outer dimensions of length 79 x 59 x 19 cm, an containment space 8 of the container of over 2700 cm² is obtained.

Such a container is suitable for use in insect breeding/ rearing, e.g. to house egg-laying mother insects, possibly in combination with a spawn structure such as according to an embodiment of the invention. In addition, the containers can be used for hatching eggs, rearing baby-larvae and the process of rearing larvae into adult insects.

It is conceivable that the container is provided with spawn structure clamps 20, to be used in combination according to an embodiment of the invention. This will be further elucidated below. The container may also be suitable for accommodating a hatch structure, e.g. a hatch structure in which parts of the spawn structure of the second aspect onto which the eggs are laid are accommodated. Possibly, the bottom of the container is at least partly removed to allow eggs or larvae to drop from the spawn structure into another container.

In embodiments, e.g. when the container is used for housing mother insects, it is possible to remove at least a part of the bottom of the container, and replace it by a mesh, e.g. for the removal of excrements.

In fig. 1a, a stack 9 of two containers according to the disclosure is shown. Advantageously, a stack comprises at least twenty containers. Preferably, a few stacks are positioned on a pallet, e.g. 2-4 stacks. Advantageously, an insect breeding facility is provided with one or more climate housings, or climate areas, in which multiple stacks of containers are provided.

The corner structure 10 according to the first aspect of the disclosure will be explained in relation to the corner structure 10 in fig. 1b between walls 4 and 5. In the shown embodiment, all four corner structures 10 have the same configuration.

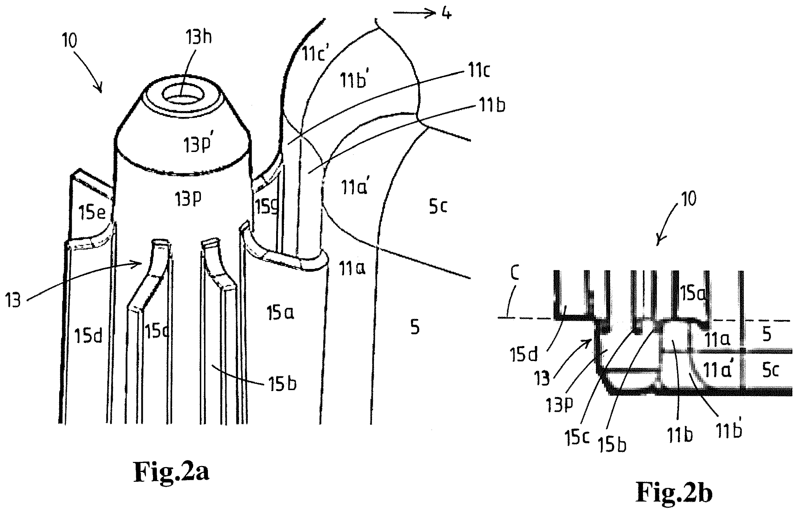

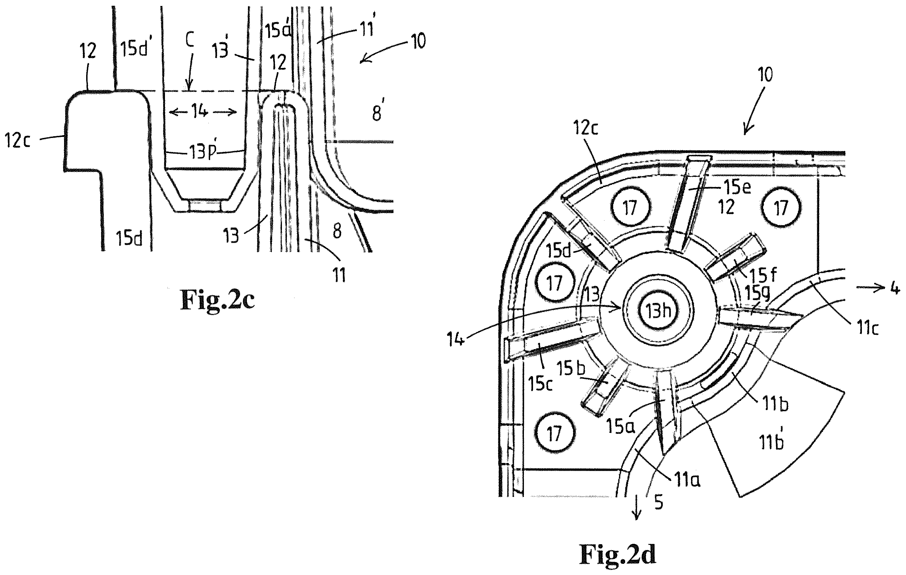

The corner structure 10 comprises a substantially diagonal and upright wall section 1 1 which, seen in top view, extends diagonally between adjoining upright walls 4,5 and blends into said upright walls to define the contour of a containment space 8 of the container. In particular, in the shown embodiment, as visible in fig. 1b and figs. 2a, 2b, and 2d, the upright wall section 1 1 comprises three portions: portion 11 an adjacent wall 5, upright wall portion 1 1 c adjacent wall 4, and upright wall portion 1 1 b therebetween.

At a bottom end, the upright wall section 11 is joined to the bottom similar to the type of join of the upright walls to the bottom, via curved sections 11 a1 , 11b1 , 11 c1.

In the shown embodiment, a top wall 12 extends outward from an upper end of the diagonal and upright wall section, to extend essentially parallel to the bottom 2.

In the shown embodiment, the first and second pairs of parallel upright walls 2,3 ; 4,5 comprise a top flange 2a, 3a; 4a,5a extending outward from an upper end of the upright walls. Here, the top flanges and the top walls 12 all extend horizontally, parallel to the bottom 2 of the container. Here, both the top flanges and the top walls 12 are provided with drainage holes 17, as visible in fig. 1b and in fig.2d.

In the shown embodiment, the top flanges 2a, 3a; 4a, 5a are further provided with flange skirt walls 2b, 3b; 4b, 5b, depending downwards from the top flanges, essentially parallel to and at a distance from the first and second pairs of parallel upright walls, and wherein preferably at least one of the skirt walls is provided with a label 18, e.g. via in-mould labelling

Here, also the corner structure 10 comprises a quarter-circular skirt wall 12c, depending downwards from an outward perimeter of the top wall 12 and at a distance from the diagonal and upright wall section.

According to the first aspect of the disclosure, a hollow tubular post 13 extends vertically downward from said top wall 12, said hollow tubular post 13 being spaced from said diagonal and upright wall section. Here the tubular post 13 is also spaced from the quarter- circular skirt wall 12c. The top wall has a hole 14 aligned with the hollow tubular post 13.

Multiple, e.g. at least three, and in the shown embodiment 7 vertical stacking ribs 15a-15g are arranged circumferentially spaced about the hollow tubular post 13, each stacking rib having a vertical inner end joined to said hollow tubular post. The stacking ribs have an exemplary thickness of 2-4 mm.

At least one, here two of said stacking ribs 15a, 15g have vertical outer end joined to said diagonal and upright wall section 1 1 a, 1 1 c respectively. It is also conceivable that the vertical outer ends are joined to a wall of the first and second pairs of parallel upright walls. Furthermore, here part of vertical outer ends of stacking ribs 15c, 15d, 15e is joined to said quarter-circular skirt wall 12c. The vertical outer end of stacking ribs 15b, 15f is not joined to any other part.

Each of the stacking ribs has an upper end. Here, the upper ends of said stacking ribs 15a- 15g join said top wall 12. Furthermore, each of the stacking ribs has a lower end. According to the first aspect of the disclosure, said lower ends are located in a common plane C. Here, the lower ends are slightly rounded, which is advantageous, as visible in fig. 2c, to match the hole 14 in which the protruding stacking pin portion is received when stacked.

The tubular post 13 has a protruding stacking pin portion 13p extending downward beyond said common plane of the stacking rib lower ends. In the shown embodiment, the protruding stacking pin portion 13p comprises a tapering end portion 13p'. Here, the bottom end of the stacking pin portion 13p is provided with a drainage hole 13h. As visible in particular in fig. 2c, in a stack the pin portion 13p' of an upper container V is received through the hole 14 in the top wall 12 of the corner structure of a lower container 1 onto which said upper container is stacked.

In the shown preferred embodiment, said stacking pin portion 13p does not protrude downward beyond the bottom 2 of the container and the lower ends of the stacking ribs in common plane C lie above the bottom so that - in a stack - the upper container nests in the lower container. In particular, the lower ends of the stacking ribs of the upper container, here ribs 15d' and 15a' are visible, rest onto the top wall 12 of the lower container, while the stacking pin portion 13p' is stacked through the hole 14 into the tubular post 13 of the lower container 1. Here, in a stack, the bottom 2' of the upper container is slightly below the level of the top walls of the corner structures of the lower container. Hence, there is an overlap between the containers 1 and 1 '. In other words, the relative distance between the containers is negative.

The length of the stacking pin portion attributes to the stability of a stack of containers. An advantageous length is 30 mm, for containers having a height of 190mm, resulting in a stacking height of a container of about 160 mm.

In embodiments (not shown) with the stacking pin portion 13p protruding downward beyond the bottom 2 of the container, the lowermost container of a stack will rest on the stacking pin portions and not on its bottom. Furthermore, the relative distance between containers may be enlarged. The relative distance between containers is determined by the location of the common plane C of the lower ends of the stacking ribs.

In embodiments (not shown) with the bottom end of the stacking pin portion 13p at a distance above the bottom 2 of the container, the lowermost container of a stack will rest on the bottom and not on the stacking pin portions. Furthermore, the relative distance between containers may be very small, such that the containers overlap to a larger extent.

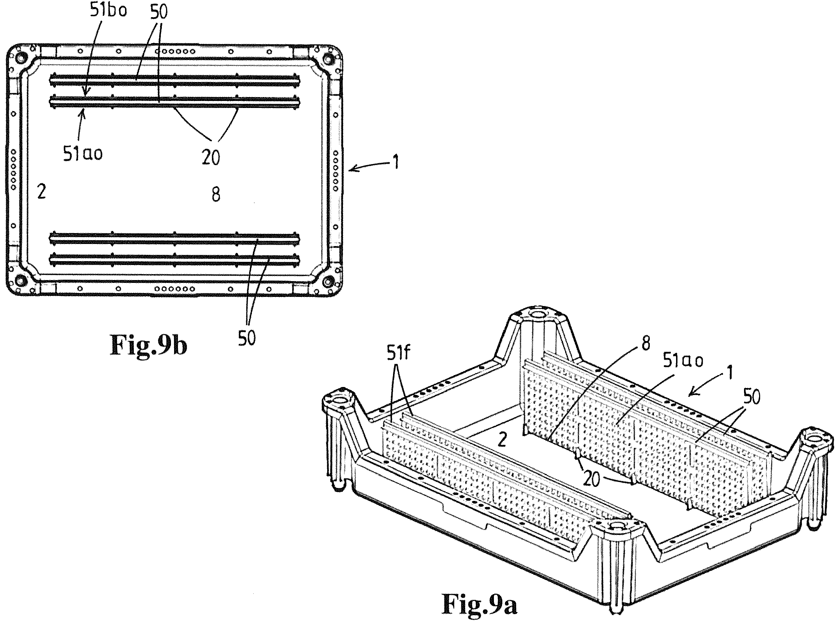

The second aspect of the disclosure, which is the subject matter of the present invention, relates to the combination of an insect spawning container 1 and at least one spawn structure 50, an example of which is shown in figs 9a and 9b. The insect spawning container 1 here corresponds to the container 1 of the first aspect of the disclosure. Alternative container configurations are also conceivable, in particular stackable containers, as long as they are suitable to be used as an insect spawning container in an insect breeding facility for breeding insects of the type with crawling mother beetles having a protractable egg-laying tube, such as lesser mealworms or zophobas morios. The insect spawning container 1 comprises a bottom 2, first and second pairs 2,3; 4,5 of parallel upright walls and a containment space 8 for the mother beetles.

A spawn structure 50, here 4 spawn structures 50, is provided in combination with the container 1 , for the mother insects to spawn their eggs. Here, the spawn structures 50 are provided in the longitudinal direction of the spawning container 1, at side parts of the container, leaving the central area between the spawn structures 50 open. In the shown configuration, the length of the spawn structure is between 70-100%, in particular 80-90%, of that of the insect spawning container. The width of a spawn structure is relatively small, in the order of 1-5 % of the width of the container, in particular in the order of 8-20 mm, in particular 10-15 mm.

In the shown embodiment, as visible in fig. 1b, fig. 9a and 9b, the bottom 2 of the container is possibly provided with spawn structure clamps 20, to receive a spawn structure in the spawning configuration and allow the spawn structure to be connected to the container 2. Preferably, this is a spawn structure according to an embodiment of the invention.

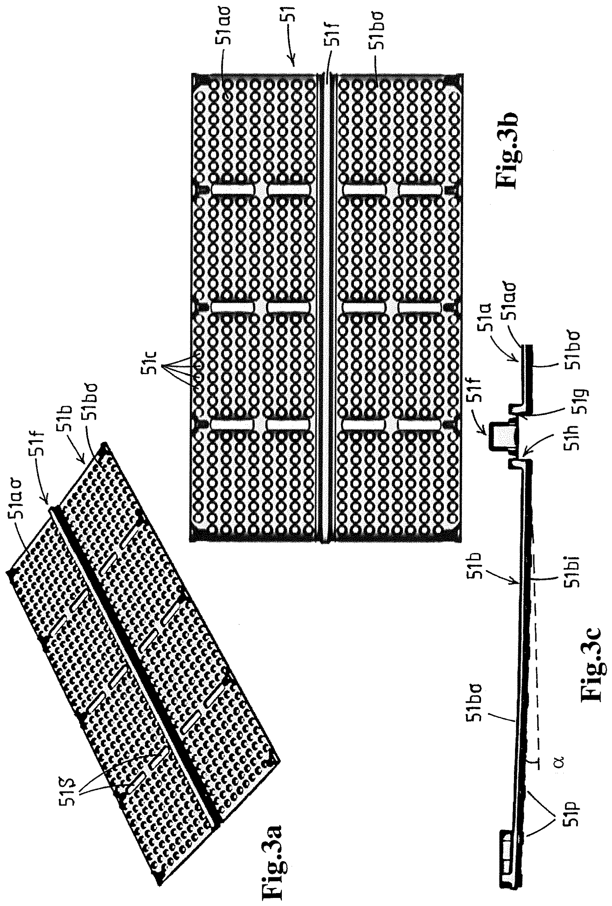

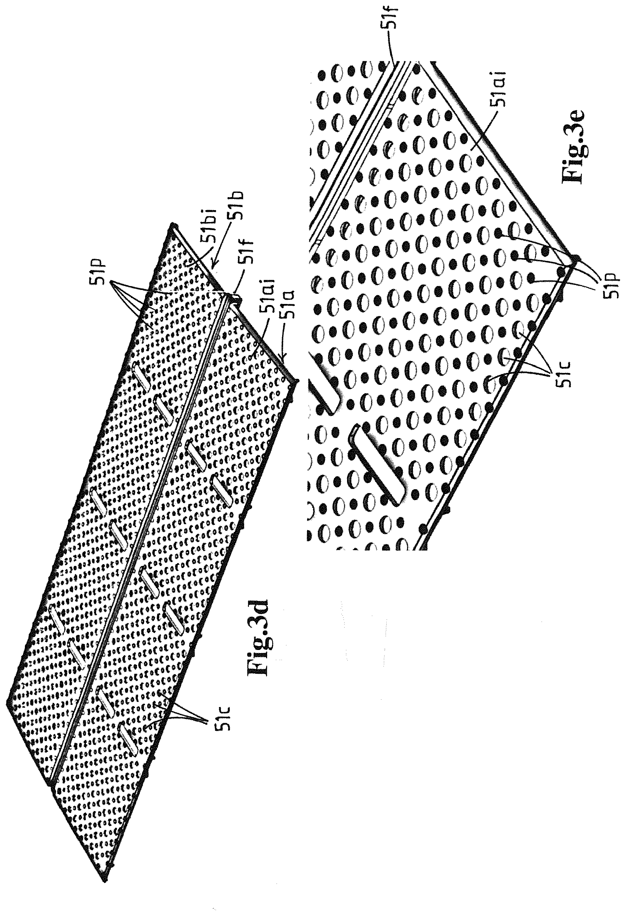

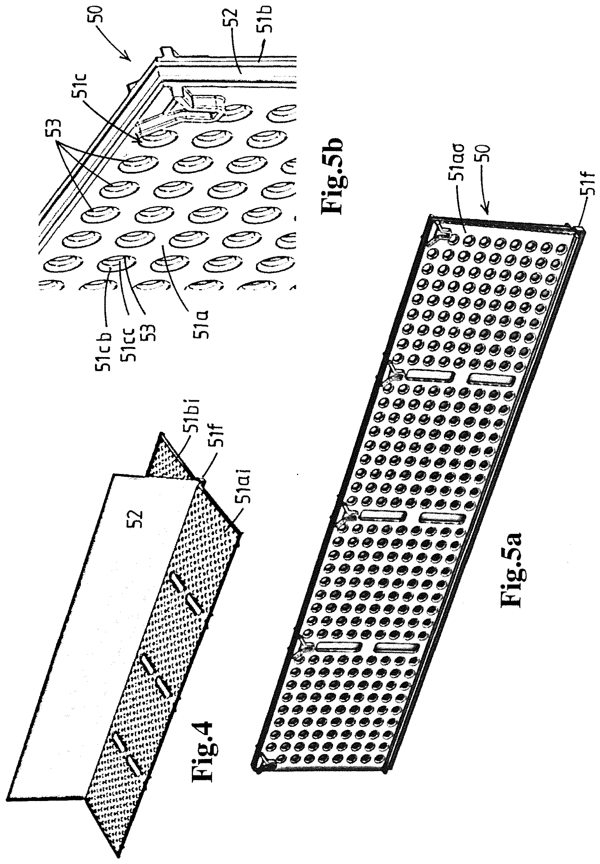

The spawn structure comprises a foldable member 51 with perforations or cut-aways 51 c, as shown in figs. 3a- 3e, and a rigid plate 52, visible in fig. 4. The foldable member 51 and the rigid plate 52 are preferably monolithically injection molded plastic products.

In figs. 3a-3e the foldable member 51 of the spawn structure according to an embodiment of the invention is shown in detail, without the rigid plate 52 of the spawn structure 50.

According to an embodiment of invention, this foldable member 51 comprises a fold section 51 f provided centrally between, and via hinges 51 g, 51 h connected to, a perforated left-hand section 51 a and a perforated right-hand section 51 b.

The left-hand section 51 a and right-hand section 51 b are embodied as plates, and are provided with perforations 51 c. In the shown configuration, the foldable member 51 comprises 8 segments, here each provided with 80-81 perforations. Each section 51 a, 51 b comprises an outer face 51ao, 51 bo and an inner face 51ai, 51 bi, respectively.

According to an embodiment of the invention, the foldable member and the rigid plate are movable with respect to each other between an open configuration and a closed spawning configuration. The mutual distance between the foldable member and rigid plate is increased and decreased during this movement, in particular the distance between the inner faces 51ai, 51bi.

In figs. 5a, 5b and 9a, 9b the spawn structure 50 is shown in a closed spawning configuration. In the spawning configuration, the foldable member 51 is folded such that the left-hand section 51 a and the right-hand section 51 b are parallel to each other. The rigid plate 52 is sandwitched between the sections 51 a, 51 b. A multitude of crevices 53 is created between the rigid plate 52 and inner faces 51 ai, 51 bi of the folded member. These crevices 53 have dimensions tuned to the egg-laying tube of the mother beetles. For breeding lesser mealworms the width of the crevices is 0,2-1 ,2 mm, while for breeding zophobas morios the width of the crevices is 0,2-1 ,8 mm. The depth of the crevices may vary between 0,5-10,0 mm.

In the spawning configuration, as visible in figs. 9a and 9b, the spawn structures 50 are positioned in the container, here adjacent the bottom 2. The outer faces 51 ao, 51 bo of the folded member 51 form two climbing faces which are positioned adjacent the bottom of the container. An advantage of the foldable member is that crevices are created at both sides of the spawn structure.

The spawn structure in the closed spawning configuration is positioned in the container such that the mother beetles are able to crawl from the bottom onto and up along the climbing faces of the spawn structure into the crevices, e.g. via the spawn structure clamps 20.

In an open configuration, as visible in fig. 4, the foldable member 51 is folded open, and the distance between the foldable member 51 and rigid plate 52 is increased, allowing harvesting of the eggs, and/ or cleaning of the spawn structure.

The configuration of the spawn structure according to the second aspect allows efficient handling of the spawn structure, e.g. to switch between the closed and the open configuration. An increase in efficiency will may attribute to an increased yield, in particular during scaling up the breeding of insects.

Furthermore, an increased yield is obtained by allowing the opening of the spawn structure by a sideways movement of the foldable member 51 , in particular of the left-hand part 51 a and the right-hand part 51 b, similar to the opening of a book. This sideways opening diminishes the risk of damaging the eggs, as no transversal relative sliding movement of the foldable member and rigid plate of the spawn structure is possible.

Also attributing to the prevention of relative sliding is providing the fold section 51 f with an elongated slit seat 51fs having a length corresponding to the length of the rigid plate, a width essentially corresponding to the thickness of the rigid plate, and a depth to accommodate the rigid plate.

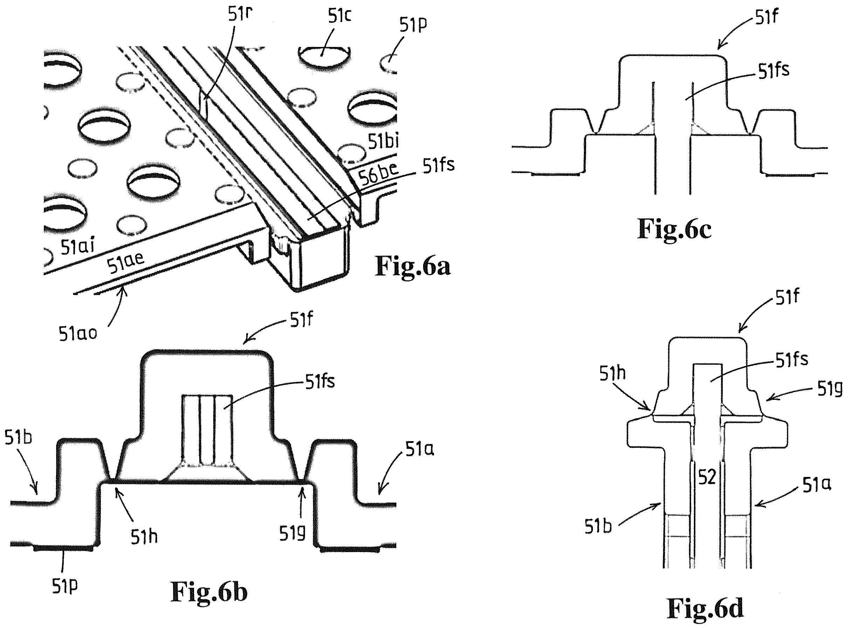

This is shown in more detail in figs. 6a-6d. In particular, in fig. 6a the 'empty' slit 51fs without the rigid plate 52 is shown. Also, an optionally provided rib 51 r in the slit 51fs is visible, which may be provided to attribute to fixation of the rigid plate 52.

In fig. 6d, the rigid plate 52 is positioned into the slit seat 51fs.

The yield correlates with the number of crevices 53 having the correct dimensions.

In particular in fig. 5b, a detail of an advantageous type perforation is visible. The perforations 51 c are circular, and here provided with a bevelled part 51 cb, such that the perforation 51 c comprises a relatively large diameter adjacent the outer surface 51 ao of the foldable plate, tapering to a smaller diameter adjacent a cylindrical part 51 cc of the perforation, which ends at the inner surface 51 ai of the foldable plate 51. The crevices 53 are formed between the end of the cylindrical part 51 cc and the rigid plate 52.

Advantageously, in the configuration as shown as can be derived in particular from fig. 4, the foldable member 51 has a length in the direction of the fold section 51 f, essentially corresponding to the length of the rigid plate 52, and wherein the width of the left-hand and right-hand sections essentially correspond to the width of the rigid plate. As a result, no additional crevices or clear spaces are created between the foldable member and rigid plate, other than the envisaged crevices.

To obtain crevices having dimensions tuned to the egg-laying tube of the mother beetles, advantageously the inner faces 51 ai, 51 bi of the foldable member are provided with protrusions 51 p, in particular visible in fig. 3e. E.g. protrusions of 0,3 - 0,4mm high are suitable to create crevices of the desired dimensions.

In view of the importance of the dimensions of the crevices, it is important to accurately control the mutual distance between the foldable member and the rigid plate in the closed spawning configuration. The provision of protrusions 51 p ensures a minimum distance between the parts of the spawn structure. On the other hand, measures may be contemplated to reduce the risk of having a too large mutual distance.

E.g. in fig. 3a, elongated savings 51s are visible between the perforated sections. These are provided to prevent bulging out of the left- and right-hand sections. Another measure to control the dimensions of the crevices is to provide a pre-stress to at least one of the hinges 51 g, 51 h between the left-hand and right-hand sections 51 a, 51 b and the fold section 51 f, opposite the folding direction.

In fig. 3c, the pre-stress is visible as a slight angle a of 1-1 ,5° between the right-hand section 51 b and the horizontal, while here the left-hand section 51 does not include an angle with the horizontal.

In fig. 6a, it is visible that sides 51 ae, 51 be of the left-hand section 51 a and right-hand section 51 b respectively of the foldable member 51 , adjacent the fold section 51 f, taper towards the outer faces 51 ao, 51 bo respectively. By allowing the ends to taper towards the outer faces, in the spawning configuration an opening is created, between the parallel left- hand section and the right-hand section on the one hand, and the rigid plate on the other hand. This opening facilitates the increasing of the distance between the foldable member and rigid plate of the spawn structure to the open configuration. Possibly, the opening allow the use of an opening tool to increase the mutual distance. The ends of the inner faces of the foldable member opposite the fold section and/or the sides of the foldable member adjacent the fold section may taper towards the outer faces.

Yet another optional measure to control the dimensions of the crevices is to ensure a proper closing of the spawn structure in the closed spawning configuration. For example, a clip is provided to close the sandwich of left-hand section 51 a, rigid plate 52 and right-hand section 51 b opposite the fold section 51 f.

In the shown embodiments, the spawn structure is positioned in the crate via spawn structure clamps 20 provided at the bottom of the insect spawning container 1. The design of these clamps 20 attributes to a proper closing of the sandwiched spawn structure 50.

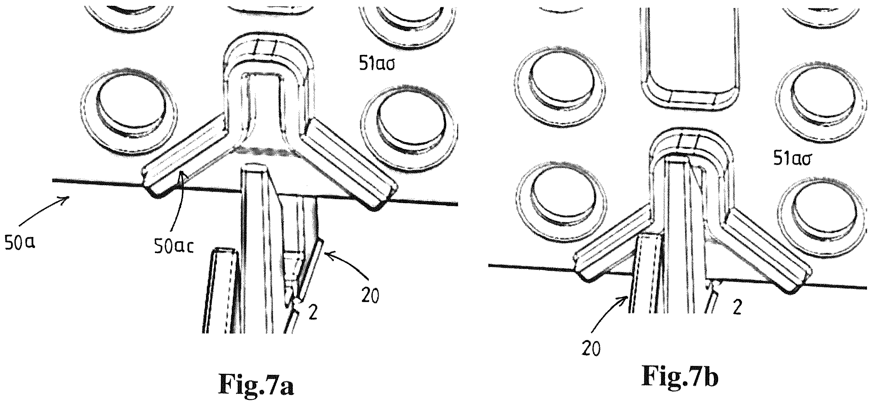

In figs. 7a-8d, the spawn structure clamp 20 is shown in further detail. The bottom 2 of the insect spawning container 1 is provided with spawn structure clamps 20 to receive the spawn structure 50 in the spawning configuration, and allow the spawn structure to be connected to the container.

In the shown embodiment, the spawn structure clamp 20 comprises opposed protruding fingers 20a, 20b, protruding from the bottom 2 of the insect spawning container, between which a spawn structure receiving groove 20c is provided. The configuration of the opposed fingers is such that the bottom of the groove between the fingers is provided at a distance from the bottom of the spawning container, e.g. 2-4, preferably 3 mm.

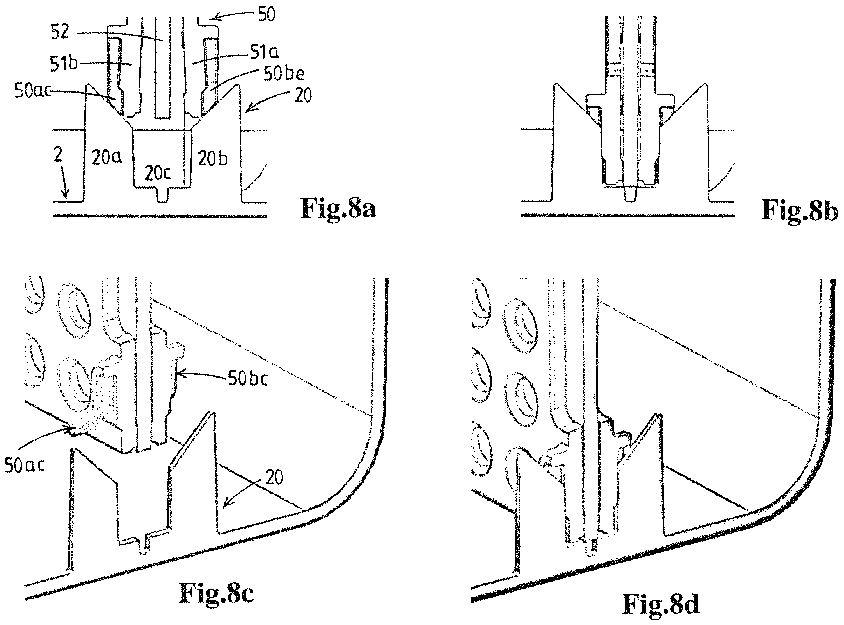

The fingers have tapering ends, to provide a guide surface 20g for the spawn structure. This is I particular visible in figs. 8a and 8b, in which the entrance of the spawn structure 50, and the closing to the closed spawning configuration is elucidated.

To define the position of the spawn structure 50 with respect to the container 1, advantageously the ends of the outer faces 50ao, 50bo of the foldable member opposite the fold section are provided with complementary container connectors 50ac, 50bc. The complementary container connectors 50ac, 50bc allow the spawn structure 50 to be connected to the container 1 in the spawning configuration. Here, the configuration of the spawn structure clamps 20 and the complementary container connectors 50ac, 50bc is such that by connecting the spawn structure to the container, the position of the spawn structure in the container is defined. Possible container connectors are snap-fitments, protruding from the outer faces.

## Claims

1. Spawn structure (50) adapted for use in an insect breeding facility for breeding insects of the type with crawling mother beetles having a protractable egg-laying tube, such as lesser mealworms or zophobas morios, in which the mother insects will spawn their eggs, **characterized in that** the spawn structure (50) comprises a foldable member (51) with a fold section (51f) provided centrally between, and via hinges (51 g, 51 h) connected to, a perforated left-hand section (51a) and a perforated right-hand section (51 b); the spawn structure further comprising a rigid plate (52);
wherein the foldable member and the rigid plate are movable with respect to each other between an open configuration and a closed spawning configuration;
wherein in the closed spawning configuration the foldable member (51) is folded such that the left-hand section (51 a) and the right-hand section are parallel to each other, with the rigid plate sandwiched between the sections (51 a, 51 b), creating a multitude of crevices (53) between the rigid plate (52) and inner faces (51 ai, 51 bi) of the folded member, the crevices having dimensions tuned to the egg-laying tube of the mother beetles; and in which spawning configuration outer faces (51 ao, 51 bo) of the folded member form two climbing faces;
and wherein in the open configuration the foldable member is folded open allowing harvesting of the eggs.

2. Spawn structure according to claim 1 , wherein the foldable member has a length in the direction of the fold section, essentially corresponding to the length of the rigid plate, and wherein the width of the left-hand and right-hand sections essentially corresponds to the width of the rigid plate.

3. Spawn structure according to claim 1 or 2, wherein the fold section (51f) comprises an elongated slit seat (51fs) having a length corresponding to the length of the rigid plate (52), a width essentially corresponding to the thickness of the rigid plate, and a depth to accommodate the rigid plate

4. Spawn structure according to any of the preceding claims 1 -3, wherein at least one of the hinges between the left-hand and right-hand sections and the fold section is pre-stressed opposite the folding direction.

5. Spawn structure according to any of the preceding claims 1 -4, wherein the ends of the inner faces of the foldable member opposite the fold section and/or the sides of the foldable member adjacent the fold section taper towards the outer faces.

6. A combination of:
- an insect spawning container (1) adapted for use in an insect breeding facility for breeding insects of the type with crawling mother beetles having a protractable egg-laying tube, such as lesser mealworms or zophobas morios; wherein the insect spawning container comprises a bottom (2) and first and second pairs (2,3; 4,5) of parallel upright walls;
- at least one spawn structure (50) according to any of the preceding claims 1-5;
wherein the spawn structure in the closed spawning configuration is positioned in the container such that the mother beetles are able to crawl from the bottom onto and up along the climbing faces of the spawn structure into the crevices.

7. Combination according claim 6, wherein the bottom (2) of the insect spawning container is provided with spawn structure clamps (20) to receive the spawn structure in the spawning configuration and allow the spawn structure to be connected to the container, and wherein preferably the ends of the outer faces of the foldable member opposite the fold section are provided with complementary container connectors.

8. Combination according to claim 6 or 7, wherein the length of the spawn structure is between 70-100%, in particular 80-90%, of that of the insect spawning container, and wherein preferably 2-10, in particular 4-6 spawn structure are provided in the longitudinal direction of the spawning container.

9. Combination according to any of the preceding claims 6-8, wherein the container is an open topped, stackable, molded plastic container (1), which is generally rectangular with a bottom (2) and first and second pairs (2,3; 4,5) of parallel upright walls joined to the bottom and at corners via corner structures (10), each corner structure comprising:
- a substantially diagonal and upright wall section (1 1) which, seen in top view, extends diagonally between adjoining upright walls and blends into said upright walls to define the contour of a containment space (8) of the container; and
- a top wall (12) extending outward from an upper end of the diagonal and upright wall section; and
- a hollow tubular post (13) extending vertically downward from said top wall (12), said hollow tubular post being spaced from said diagonal and upright wall section (1 1), wherein the top wall has a hole (14) aligned with the hollow tubular post (13);
- wherein multiple, e.g. at least three, vertical stacking ribs (15a-15g) are arranged circumferentially spaced about the hollow tubular post, each stacking rib having a vertical inner end joined to said hollow tubular post,
- wherein at least one, preferably two, of said stacking ribs (15a, 15g) have a vertical outer end joined to said diagonal and upright wall section (1 1 ) and/or to said adjoining upright wall section; and
- wherein each of said stacking ribs has a lower end, said lower ends being located in a common plane (C);
wherein said tubular post has a protruding stacking pin portion (13p) extending downward beyond said common plane of the stacking rib lower ends; such that - in a stack - the pin portion of an upper container is received through the hole in the top wall of the corner structure of a lower container onto which said upper container is stacked.

## Patentansprüche

1. Laichstruktur (50), die für die Verwendung in einer Insektenzuchtanlage zum Züchten von Insekten vom Typ mit kriechenden Mutterkäfern, die eine einziehbare Eiablageröhre haben, wie z. B. kleinere Mehlwürmer oder Große Schwarzkäfer, ausgelegt ist, in der die Mutterinsekten ihre Eier ablaichen, **dadurch gekennzeichnet, dass** die Laichstruktur (50) ein klappbares Element (51) mit einem Klappabschnitt (51f) umfasst, der mittig zwischen einem perforierten linken Abschnitt (51a) und einem perforierten rechten Abschnitt (51b) vorgesehen und über Scharniere (51g, 51h) mit diesen verbunden ist; wobei die Laichstruktur ferner eine starre Platte (52) umfasst;
wobei das klappbare Element und die starre Platte in Bezug zueinander zwischen einer offenen Konfiguration und einer geschlossenen Laichkonfiguration bewegbar sind;
wobei in der geschlossenen Laichkonfiguration das klappbare Element (51) so geklappt ist, dass der linke Abschnitt (51a) und der rechte Abschnitt parallel zueinander sind, wobei die starre Platte zwischen den Abschnitten (51a, 51b) eine Vielzahl von Spalten (53) zwischen der starren Platte (52) und den Innenflächen (51ai, 51bi) des geklappten Elements erzeugt, wobei die Spalten Abmessungen aufweisen, die auf die Eiablageröhre der Mutterkäfer abgestimmt sind; und wobei in dieser Laichkonfiguration die Außenflächen (51ao, 51bo) des umgeklappten Elements zwei Kletterflächen bilden;
und wobei in der offenen Konfiguration das klappbare Element aufgeklappt ist, was das Entnehmen der Eier ermöglicht.

2. Laichstruktur nach Anspruch 1, wobei das klappbare Element in Richtung des Klappabschnitts eine Länge aufweist, die im Wesentlichen der Länge der starren Platte entspricht, und wobei die Breite des linken und des rechten Abschnitts im Wesentlichen der Breite der starren Platte entspricht.

3. Laichstruktur nach Anspruch 1 oder 2, wobei der Klappabschnitt (51f) einen länglichen Schlitzsitz (51fs) umfasst, der eine Länge, die der Länge der starren Platte (52) entspricht, eine Breite, die im Wesentlichen der Dicke der starren Platte entspricht, und eine Tiefe zum Aufnehmen der starren Platte aufweist.

4. Laichstruktur nach einem der vorhergehenden Ansprüche 1-3, wobei mindestens eines der Scharniere zwischen dem linken und dem rechten Abschnitt und dem Klappabschnitt entgegen der Klapprichtung vorgespannt ist.

5. Laichstruktur nach einem der vorhergehenden Ansprüche 1-4, wobei sich die Enden der Innenflächen des klappbaren Elements gegenüber dem Klappabschnitt und/oder die Seiten des klappbaren Elements neben dem Klappabschnitt zu den Außenflächen hin verjüngen.

6. Kombination aus:
- einem Insektenlaichbehälter (1), der für die Verwendung in einer Insektenzuchtanlage zum Züchten von Insekten vom Typ mit kriechenden Mutterkäfern, die eine einziehbare Eiablageröhre haben, wie z. B. kleinere Mehlwürmer oder Große Schwarzkäfer, ausgelegt ist; wobei der Insektenlaichbehälter einen Boden (2) und ein erstes und ein zweites Paar (2,3; 4,5) aus parallelen aufrechten Wänden umfasst;
- mindestens eine Laichstruktur (50) nach einem der vorhergehenden Ansprüche 1-5; wobei die Laichstruktur in der geschlossenen Laichkonfiguration derart im Behälter positioniert ist, dass die Mutterkäfer vom Boden auf die Kletterflächen der Laichstruktur und entlang dieser nach oben in die Spalten kriechen können.

7. Kombination nach Anspruch 6, wobei der Boden (2) des Insektenlaichbehälters mit Laichstrukturklemmen (20) versehen ist, um die Laichstruktur in der Laichkonfiguration aufzunehmen und zu ermöglichen, dass die Laichstruktur mit dem Behälter verbunden wird, und wobei vorzugsweise die Enden der Außenflächen des klappbaren Elements gegenüber dem Klappabschnitt mit komplementären Behälterverbindungselementen versehen sind.

8. Kombination nach Anspruch 6 oder 7, wobei die Länge der Laichstruktur zwischen 70-100 %, insbesondere 80-90 %, derjenigen des Insektenlaichbehälters beträgt, und wobei vorzugsweise 2-10, insbesondere 4-6, Laichstrukturen in Längsrichtung des Laichbehälters vorgesehen sind.

9. Kombination nach einem der vorhergehenden Ansprüche 6-8, wobei der Behälter ein oben offener, stapelbarer, geformter Kunststoffbehälter (1) ist, der im Allgemeinen rechteckig mit einem Boden (2) und einem ersten und einem zweiten Paar (2,3; 4,5) aus parallelen aufrechten Wänden ist, die mit dem Boden und an den Ecken über Eckstrukturen (10) verbunden sind, wobei jede Eckstruktur Folgendes umfasst:
- einen im Wesentlichen diagonalen und aufrechten Wandabschnitt (11), der sich in der Draufsicht diagonal zwischen angrenzenden aufrechten Wänden erstreckt und in die aufrechten Wände übergeht, um die Kontur eines Aufnahmeraums (8) des Behälters zu definieren; und
- eine obere Wand (12), die sich von einem oberen Ende des diagonalen und aufrechten Wandabschnitts nach außen erstreckt; und
- einen hohlen rohrförmigen Pfosten (13), der sich von der oberen Wand (12) vertikal nach unten erstreckt, wobei der hohle rohrförmige Pfosten von dem diagonalen und aufrechten Wandabschnitt (11) beabstandet ist, wobei die obere Wand ein Loch (14) aufweist, das mit dem hohlen rohrförmigen Pfosten (13) ausgerichtet ist;
- wobei mehrere, z. B. mindestens drei, vertikale Stapelrippen (15a-15g) in Umfangsrichtung beabstandet um den hohlen rohrförmigen Pfosten angeordnet sind, wobei jede Stapelrippe ein vertikales inneres Ende aufweist, das mit dem hohlen rohrförmigen Pfosten verbunden ist,
-- wobei mindestens eine, vorzugsweise zwei der Stapelrippen (15a, 15g) ein vertikales äußeres Ende aufweisen, das mit dem diagonalen und aufrechten Wandabschnitt (11) und/oder dem angrenzenden aufrechten Wandabschnitt verbunden ist; und
-- wobei jede der Stapelrippen ein unteres Ende aufweist, wobei die unteren Enden in einer gemeinsamen Ebene (C) liegen;
wobei der rohrförmige Pfosten einen vorstehenden Stapelstiftabschnitt (13p) aufweist, der sich nach unten über die gemeinsame Ebene der unteren Enden der Stapelrippe hinaus erstreckt; so dass - in einem Stapel - der Stiftabschnitt eines oberen Behälters durch das Loch in der oberen Wand der Eckstruktur eines unteren Behälters aufgenommen wird, auf den der obere Behälter gestapelt wird.

## Revendications

1. Structure de reproduction (50) adaptée pour son utilisation dans une installation d'élevage d'insectes pour élever des insectes du type coléoptères avec mères rampantes ayant un tube de ponte déployable, comme des alphitobius diaperinus ou des zophobas morios, dans lequel les insectes mères pondront leurs oeufs, **caractérisée en ce que** la structure de reproduction (50) comprend un élément repliable (51) avec une section de pliage (51f) prévue au milieu de, et via des charnières (51 g, 51 h) raccordées à, une section gauche perforée (51a) et une section droite perforée (51 b) ; la structure de reproduction comprenant en outre une plaque rigide (52) ;
dans laquelle l'élément repliable et la plaque rigide sont mobiles l'un par rapport à l'autre entre une configuration ouverte et une configuration de reproduction fermée ;
dans laquelle, dans la configuration de reproduction fermée, l'élément repliable (51) est replié de sorte que la section gauche (51 a) et la section droite soient parallèles l'une à l'autre, avec la plaque rigide prise en sandwich entre les sections (51 a, 51 b), en créant une multitude de crevasses (53) entre la plaque rigide (52) et des faces intérieures (51 ai, 51 bi) de l'élément replié, les crevasses présentant des dimensions accordées aux tubes de ponte des coléoptères mères ; et dans laquelle configuration de reproduction, des faces extérieures (51 ao, 51 bo) de l'élément replié forment deux faces pour grimper ;
et dans laquelle, dans la configuration ouverte, l'élément repliable est ouvert pour permettre le prélèvement des œufs.

2. Structure de reproduction selon la revendication 1, dans laquelle l'élément repliable présente une longueur dans la direction de la section de pliage, correspondant sensiblement à la longueur de la plaque rigide, et dans laquelle la largeur des sections gauche et droite correspond sensiblement à la largeur de la plaque rigide.

3. Structure de reproduction selon la revendication 1 ou 2, dans laquelle la section de pliage (51f) comprend un siège à fente allongée (51fs) présentant une longueur correspondant à la longueur de la plaque rigide (52), une largeur correspondant sensiblement à l'épaisseur de la plaque rigide, et une profondeur pour loger la plaque rigide.

4. Structure de reproduction selon l'une quelconque des revendications 1 à 3, dans laquelle au moins l'une des charnières entre les sections gauche et droite et la section de pliage est précontrainte à l'opposé de la direction de pliage.

5. Structure de reproduction selon l'une quelconque des revendications 1 à 4, dans laquelle les extrémités des faces intérieures de l'élément repliable à l'opposé de la section de pliage et/ou les côtés de l'élément repliable adjacents à la section de pliage s'effilent vers les faces extérieures.

6. Combinaison :
- d'un récipient de reproduction d'insectes (1) adapté pour son utilisation dans une installation d'élevage d'insectes pour élever des insectes du type coléoptères avec mères rampantes ayant un tube de ponte déployable, comme des petits ténébrions mats ou des vers de farine géants ; dans laquelle le récipient de reproduction d'insectes comprend une partie inférieure (2) et des première et seconde paires (2,3 ; 4,5) de parois verticales parallèles ;
- d'au moins une structure de reproduction (50) selon l'une quelconque des revendications 1 à 5 ;
dans laquelle la structure de reproduction dans la configuration de reproduction fermée est positionnée dans le récipient de sorte que les coléoptères mères puissent ramper depuis la partie inférieure sur et le long des faces pour grimper de la structure de reproduction dans les crevasses.

7. Combinaison selon la revendication 6, dans laquelle la partie inférieure (2) du récipient de reproduction d'insectes est pourvue de fixations de structure de reproduction (20) pour recevoir la structure de reproduction dans la configuration de reproduction et permettre à la structure de reproduction d'être raccordée au récipient, et dans laquelle de préférence les extrémités des faces extérieures de l'élément repliable à l'opposé de la section de pliage sont pourvues de raccords de récipient complémentaires.

8. Combinaison selon la revendication 6 ou 7, dans laquelle la longueur de la structure de reproduction est entre 70 et 100 %, en particulier entre 80 et 90 %, de celle du récipient de reproduction d'insectes, et dans laquelle de préférence de 2 à 10, en particulier de 4 à 6, structures de reproduction sont prévues dans la direction longitudinale du récipient de reproduction.

9. Combinaison selon l'une quelconque des revendications 6 à 8, dans laquelle le récipient est un récipient en plastique moulé empilable à sommet ouvert (1), qui est généralement rectangulaire avec une partie inférieure (2) et des première et seconde paires (2,3 ; 4,5) de parois verticales parallèles jointes à la partie inférieure et à des coins via des structures de coin (10), chaque structure de coin comprenant :
- une section de paroi verticale sensiblement diagonale (1 1) qui, en vue de dessus, s'étend diagonalement entre des parois verticales contiguës et s'intègre dans lesdites parois verticales pour définir le contour d'un espace de confinement (8) du récipient ; et
- une paroi supérieure (12) s'étendant vers l'extérieur depuis une extrémité supérieure de la section de paroi verticale diagonale ; et
- un montant tubulaire creux (13) s'étendant verticalement vers le bas depuis ladite paroi supérieure (12), ledit montant tubulaire creux étant espacé de ladite section de paroi verticale diagonale (1 1), dans laquelle la paroi supérieure comprend un trou (14) aligné sur le montant tubulaire creux (13) ;
- dans laquelle plusieurs, par exemple au moins trois, nervures d'empilement verticales (15a-15g) sont agencées circonférentiellement autour du montant tubulaire creux, chaque nervure d'empilement ayant une extrémité intérieure verticale jointe audit montant tubulaire creux,
- dans laquelle au moins l'une, de préférence deux, desdites nervures d'empilement (15a, 15g) a une extrémité extérieure verticale jointe à ladite section de paroi verticale diagonale (1 1) et/ou à ladite section de paroi verticale contiguë ; et
- dans laquelle chacune desdites nervures d'empilement a une extrémité inférieure, lesdites extrémités inférieures étant situées dans un plan commun (C) ;
dans laquelle ledit montant tubulaire comporte une partie de broche d'empilement saillante (13p) s'étendant vers le bas au-delà dudit plan commun des extrémités inférieures de nervures d'empilement ; de sorte que, dans un empilement, la partie de broche d'un récipient supérieur soit reçue à travers le trou dans la paroi supérieure de la structure de coin d'un récipient inférieur sur lequel ledit récipient supérieur est empilé.
